# EUROPÄISCHE PATENTSCHRIFT

(11) **EP 0 444 563 B1**
(45) Veröffentlichungstag und Bekanntmachung des Hinweises auf die Patenterteilung: **23.11.1994**
(21) Anmeldenummer: 91102714.2
(22) Anmeldetag: 25.02.1991
(51) Int. Cl.: C07C 49/707, C07C 45/67, C07C 45/68, C07C 45/64, C07C 45/63, C07C 45/43, C07C 211/05, C07C 211/35

(54) **3-Hydroxy-2-cyclobuten-1-on-salze, deren Herstellung und Verwendung**
3-Hydroxy-2-cyclobuten-1-on salts, their preparation and use
Sels de 3-hydroxy-2-cyclobutène-1-on, leur préparation et utilisation

(30) Priorität: 26.02.1990 CH 598/90
(43) Veröffentlichungstag der Anmeldung: 04.09.1991
(73) Patentinhaber: LONZA AG, CH-3945 Gampel/Wallis (CH)
(72) Erfinder: Jackson, Barry, Dr., Glis, Kanton Wallis (CH); Scholl, Thomas, Dr., Visp, Kanton Wallis (CH)
(74) Vertreter: Weinhold, Peter, Dr.

(56) Entgegenhaltungen:
- US-A- 3 244 749
- US-A- 3 379 746

## Beschreibung

Die Erfindung betrifft neue 3-Hydroxy-2-cyclobuten-1-on-Salze gemäss Formel I sowie Verfahren zu deren Herstellung und deren Verwendung, insbesondere zur Herstellung von Quadratsäure.

Die neuen 3-Hydroxy-2-cyclobuten-1-on-salze gemäss der allgemeinen Formel I sind wertvolle, stabile Substanzen. Einerseits sind sie Aequivalente für das bei Raumtemperatur sich zersetzende, oder synthetische interessante 1,3-Cyclobutandion, andererseits sind sie Zwischenprodukte einer neuen Synthese von Quadratsäure.

Quadratsäure ist ein interessantes Zwischenprodukt zur Herstellung von Pharmazeutika, Farbstoffen (Angew. Chem., 20, 1966, S.931) und Herbiziden (CH-PS 609 837).

Aus der Literatur sind verschiedene Verfahren zur Herstellung von Quadratsäure bekannt.

Mehrere gehen von Hexachlor-1,3-butadien aus, das mit Hilfe von Natriumethanolat zu einem chlorierten Cyclobuten-Derivat zyklisiert wird. Diese Zwischenprodukte werden mit Schwefelsäure oder anderen Säuren zu Quadratsäure hydrolysiert (Roedig, A. und Bernemann P., Liebigs Ann. Chem. 1956, 600, S.1; Maahs, G., Liebigs Ann. Chem. 1965, 686, S.55; Angew. Chemie, 1963, 75, S.982; Uehara, A. und Tsuchiya, R., Sci. Rep. Kanazawa Univ. 1980, 25, S.83; Fan, R. et al., Chemical Abstracts 1987, 106, 103798c). Statt Natriumethanolat wird auch Morpholin verwendet (Maahs, G. und Hegenberg P., Angew. Chemie 1966, 78, S.927; Schmidt, A.H. und Ried., W., Synthesis 1978, S.869, Gadek, T.R. et al. 1976, US-PS 4 104 308; Paine, A.J., Tetrahedron Letters, 1984, 25, S.135). Die Zyklisierung kann auch rein thermisch erfolgen (Müller, W. 1976, DE-PS 26 18 557; Schröder, M. und Schäfer, W. 1976, DE-PS 26 23 836; Maahs, G. und Rombusch, D. 1978, DE-PS 28 24 558; Rombusch, K. und Maahs, G. 1983 DE-OS 33 14 431).

Nachteile all dieser Verfahren sind entweder bescheidene Ausbeuten oder hoher Aufwand (z.B. Destillation mit extremem Rücklaufverhältnis) und die besonderen Sicherheitsmassnahmen, die beim Umgang mit dem krebserzeugenden Edukt Hexachlor-1,3-butadien erforderlich sind.

Nach einem anderen Verfahren (Bellus, D. et al., Helv. Chim. Acta 61, 1978, S.1784) wird aus dem Pilzmetaboliten Moniliformin durch Bromierung und Hydrolyse Quadratsäure in 70% Ausbeute gewonnen. Moniliformin kommt aber in der Natur nur in kleinen Mengen vor, und die bekannten Synthesen dafür sind aufwendig und ergeben nur bescheidene Ausbeuten.

Ein weiteres Verfahren, die elektrochemische reduktive Tetramerisierung von Kohlenmonoxid zu Quadratsäure, verlangt einen grossen apparativen Aufwand und ergibt ein Produktegemisch, aus dem Quadratsäure nur schwer in reiner Form erhältlich ist. (Silvestri, G. et al., Gazz. Chim. It. 1972, 102, S.818; DE-OS 22 35 882; US-PS 4 461 681, US-PS 4 523 980, Fabre P.L. et al., Bull. Soc. Chim. Fr. 1988, S.933)

Aufgabe der vorliegenden Erfindung war es, eine neue Synthese von Quadratsäure vorzuschlagen, die von leicht zugänglichen Edukten aus mit bescheidenem Aufwand Quadratsäure in guter Ausbeute und Reinheit ergibt.

Die Aufgabe der vorliegenden Erfindung wurde gelöst durch das Auffinden neuer Zwischenprodukte, die von einem leicht zugänglichen Edukt hergestellt werden können und aus denen Quadratsäure ohne hohen Aufwand hergestellt werden kann.

Die erfindungsgemässen Zwischenprodukte haben die allgemeine Formel 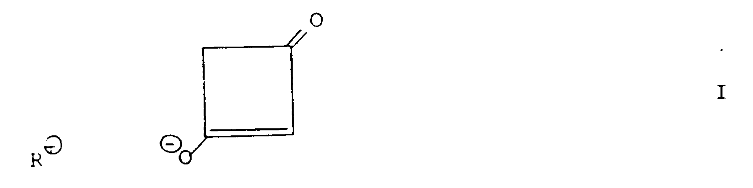
worin R entweder eine Ammonium-Gruppe der allgemeinen Formel 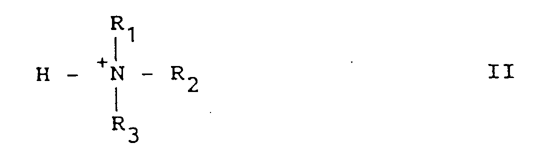
in der R₁, R₂ und R₃ gleichbedeutend oder verschieden sind und ein Wasserstoffatom, eine C₁-C₄-Niederalkyl- oder eine Cycloalkylgruppe darstellt, oder R ein Alkalimetallatom bedeuten.
Die bevorzugten Reste R₁, R₂ und R₃ der Ammonium-Gruppe sind ein Wasserstoffatom, eine C₁ - C₄-Niederalkyl- oder eine Cyclohexylgruppe, insbesondere ein Wasserstoffatom, eine Methyl- oder Ethylgruppe oder eine Cyclohexylgruppe.
Wenn R ein Alkalimetallatom bedeutet, sind die Alkalimetalle Natrium und Kalium bevorzugt.

Die bevorzugten Verbindungen der allgemeinen Formel I sind:
3-Hydroxy-2-cyclobuten-1-on-diethylammoniumsalz
3-Hydroxy-2-cyclobuten-1-on-cyclohexylammoniumsalz
3-Hydroxy-2-cyclobuten-1-on-cyclohexyldimethylammoniumsalz
3-Hydroxy-2-cyclobuten-1-on-dicyclohexylammoniumsalz
3-Hydroxy-2-cyclobuten-1-on-dicyclohexylmethylammoniumsalz
3-Hydroxy-2-cyclobuten-1-on-ammoniumsalz
3-Hydroxy-2-cyclobuten-1-on-natriumsalz
3-Hydroxy-2-cyclobuten-1-on-kaliumsalz

Das für das Verfahren der Erfindung verwendete Ausgangsmaterial ist 3-Acetoxy-2-cyclobuten-1-on, das im folgenden als Triketen bezeichnet wird, gegebenenfalls ein Destillationsrückstand der Diketen-Produktion mit einem Triketen-Gehalt von zweckmässig 5 bis 60 Gew.%, oder 1,3-Cyclobutandion.
Diese genannten Ausgangsmaterialien werden mit einer Base zum Endprodukt gemäss Formel I umgesetzt. Als Base wird zweckmässig entweder ein Amin der allgemeinen Formel 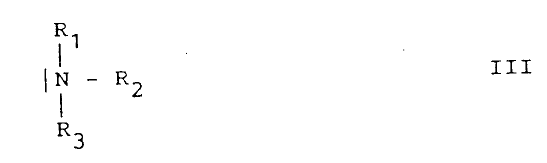
in der R₁,R₂ und R₃ die genannte Bedeutung haben, oder ein Alkalimetallalkoholat oder Alkalimetallhydroxid eingesetzt. Vorzugsweise werden als Amine der allgemeinen Formel III Cyclohexyldimethylamin, Dicyclohexylamin, Diethylamin, Cyclohexyldimethylamin, Dicyclohexylmethylamin oder Ammoniak eingesetzt.

Die Umsetzung von Triketen als Ausgangsmaterial wird zweckmässig mit 0,5 bis 4 Molen eines Amins der allgemeinen Formel III, bezogen auf 1 Mol Triketen, durchgeführt.

Die Umsetzung von 1,3-Cyclobutandion als Ausgangsmaterial wird zweckmässig mit 0,5 bis 4 Molen eines Amins der allgemeinen Formel III, vorzugsweise mit 0,5 bis 1,5 Molen, bezogen auf 1 Mol 1,3-Cyclobutandion, durchgeführt.

Die Reaktion wird üblicherweise bei einer Temperatur von -40 bis 50°C, vorzugsweise von 10 bis 25°C durchgeführt. Nach einer Reaktionszeit von 15 Minuten bis 5 Stunden, kann das Endprodukt gemäss Formel I durch übliche Aufarbeitung, z.B. durch eine Filtration, erhalten werden.

Als Lösungsmittel können niedrig siedende aliphatische Alkohole, C₁ - C₄-Carbonsäuren, C₁ - C₄-Carbonsäureester, niedrig siedende Ether und Ketone, Nitrile und aromatische Kohlenwasserstoffe, angewendet werden. Vertreter dieser Lösungsmittelgruppen sind beispielsweise Aceton, Toluol, Diethylether, Diisopropylether, t-Butylmethylether und Dichlormethan, Ethanol, Acetonitril, Essigsäure und Essigsäureethylester; vorzugsweise wird Essigsäureethylester, Ethanol oder Acetonitril angewendet.

Die 3-Hydroxy-2-cyclobuten-1-on-salze gemäss Formel I können auch durch Umsetzung der Ausgangsmaterialien mit 0,8 bis 2,5 Molen eines Alkalimetallalkoholats oder eines Alkalimetallhydroxids, wie beispielsweise mit Natrium- oder Kaliumhydroxid, Natrium- oder Kaliumethanolat, Natrium- oder Kaliummethanolat, bezogen auf 1 Mol Ausgangsmaterial, vorzugsweise mit 0,8 bis 1,2 Molen Natriumhydroxid, Kaliumhydroxid, Natriummethanolat oder Natriumethanolat hergestellt werden.

Die Reaktion wird zweckmässig, wie die Umsetzung des Ausgangsmaterials mit dem Amin gemäss Formel III, bei einer Temperatur von -40 bis 50°C, vorzugsweise von 10 bis 25°C durchgeführt. Nach einer Reaktionszeit von 15 Minuten bis 5 Stunden kann das Endprodukt gemäss Formel I durch übliche Aufarbeitung, z.B. Filtration, erhalten werden.

Als Lösungsmittel können die gleichen wie die zuvor beschriebenen angewendet werden.

Die neuen 3-Hydroxy-2-cyclobuten-1-on-salze können auch in zwei Stufen hergestellt werden, indem man in einer ersten Stufe Triketen durch saure Hydrolyse in 1,3-Cyclobutandion überführt und dieses Zwischenprodukt dann in einer zweiten Stufe in das 3-Hydroxy-2-cyclobuten-1-on-salz, wie schon beschrieben, überführt.
Als Säure können Schwefelsäure, Salzsäure, Ameisensäure, Trifluoressigsäure angewendet werden, vorzugsweise wird wässrige Ameisensäure im Ueberschuss angewendet.
Die Hydrolyse wird üblicherweise bei einer Temperatur von 0 bis 30°C, vorzugsweise von 15 bis 30°C, durchgeführt.
Nach einer Reaktionszeit von in der Regel 15 Minuten bis 24 Stunden, kann das 1,3-Cyclobutandion auf übliche Art und Weise, zweckmässig durch Extraktion und Umkristallisation, aufgearbeitet werden.

Die neuen 3-Hydroxy-2-cyclobuten-1-on-salze gemäss Formel I sind geeignet zur Herstellung von Quadratsäure, wobei Quadratsäure in guter Ausbeute und Reinheit isoliert wird, oder sie können wieder zur Herstellung von 1,3-Cyclobutandion eingesetzt werden.

Zur Herstellung von Quadratsäure werden die 3-Hydroxy-2-cyclobuten-1-on-salze gemäss Formel I in einer ersten Stufe halogeniert und dann in einer zweiten Stufe zur Quadratsäure hydrolysiert.

Die erste Stufe, die Halogenierung von 3-Hydroxy-2-cyclobuten-1-on-salz, wird zweckmässig mit 2 bis 4 Molen Brom, Chlor oder Sulfurylchlorid bezogen auf 1 Mol Ausgangsmaterial, vorzugsweise mit 2,5 bis 3,5 Molen Brom, durchgeführt.
Die Halogenierung kann auch zuerst mit 0,25 bis 1 Mol Brom und anschliessend mit 2,0 bis 3,0 Molen Chlor oder Sulfurylchlorid, bezogen auf 1 Mol Ausgangsmaterial, durchgeführt werden.

Die Halogenierung wird üblicherweise bei einer Temperatur von -40 bis +40°C, vorzugsweise von -25 bis 25°C, durchgeführt. Nach einer Reaktionszeit von zweckmässig 30 Minuten bis 4 Stunden kann das halogenierte Cyclobutenon in guter Ausbeute isoliert oder direkt weiter zur Quadratsäure hydrolysiert werden. Als Lösungsmittel können C₁ - C₄-Carbonsäuren, C₁ - C₄-Carbonsäureethylester, Carbonsäureanhydride und chlorierte Kohlenwasserstoffe angewendet werden. Vertreter dieser Lösungsmittelgruppen sind beispielsweise Essigsaure, Essigsäure-ethyleser,Acetanhydrid, Tetrachlorkohlenstoff, Methylenchlorid, Chloroform, vorzugsweise setzt man Essigsäure, Essigsäureethylester oder Methylenchlorid ein.

Die zweite Stufe, die Hydrolyse zur Quadratsäure, kann mit Mineralsäuren, wie beispielsweise Schwefelsäure, Salzsäure, Bromwasserstoffsäure, Phosphorsäure, mit Sulfonsäuren, wie beispielsweise wässriger Methansulfonsäure, mit Wasser oder mit Carbonsäuren, wie beispielsweise wässriger Ameisensaure und wässriger Trifluoressigsäure, durchgeführt werden. Vorzugsweise werden Mineralsäuren, wie beispielsweise konzentrierte Schwefelsäure oder Salzsäure, Carbonsäuren, wie beispielsweise wässrige Ameisensäure, Sulfonsäuren, wie beispielsweise wässrige Methansulfonsäure, im Ueberschuss eingesetzt.
Die Hydrolyse wird zweckmässig bei einer Temperatur von 50 bis 150°C, vorzugsweise bei einer Temperatur von 90 bis 100°C, durchgeführt. Die Hydrolyse zur Quadratsäure mit Wasser wird unter Rückfluss durchgeführt. Nach einer Reaktionszeit von 2 bis 48 Stunden wird Quadratsaure in guter Ausbeute, erhalten.

### Beispiel 1

### Herstellung von 1,3-Cyclobutandion ausgehend von Triketen

4,1 g Triketen (33 mMol) wurden in 10 g Schwefelsäure und 30 g Eis gelöst. Nach 15 Minuten wurde das Gemisch mit Methylenchlorid (2 mal je 25 ml) extrahiert, dann über Calciumchlorid getrocknet, anschliessend filtriert und eingeengt. 2,1 g des eingeengten, festen Materials wurden in Diethylether (10 ml) aufgeschlämmt, anschliessend wurde die Mischung filtriert und dann in Acetonitril (13 ml) umkristallisiert. Nach der Umkristallisation wurden 1,57 g 1,3-Cyclobutandion (18,7 mMol), entsprechend einer Ausbeute von 57%, bezogen auf eingesetztes Triketen, erhalten.

### Beispiel 2

### Herstellung von 3-Hydroxy-2-cyclobuten-1-on-diethylammoniumsalz

a) In 40 g Essigester wurden 2,1 g 1,3-Cyclobutandion (23,0 mMol) suspendiert. Zu dieser Suspension wurde bei 20°C innerhalb 15 Min. eine Lösung von 1,84 g Diethylamin (25,0 mMol) in 10 g Essigsäureethylester zugetropft.
   Nach 1 Stunde Rühren bei Raumtemperatur wurde die Suspension abfiltriert und anschliessend getrocknet. Es wurden 3,05 g des Titelproduktes mit einer Reinheit (nach HPLC) von 93,7%, entsprechend einer Ausbeute von 79,2%, bezogen auf Cyclobutandion, erhalten.
   Smp: 93°C - 94°C
b) Zu einer Lösung von 3,25 g 3-Acetoxy-2-cyclobutenon-1-on (Triketen, Gehalt, 97%; 25,0 mMol) in 40 g Essigsäure-ethylester, wurde bei 20°C innerhalb von 18 Min. eine Lösung von 3,67 g Diethylamin (99,5%; 50,0 mMol) in 10 g Essigester zugetropft. Das Reaktionsgemisch wurde noch 1 Stunde bei Raumtemperatur gerührt, dann abfiltriert und unter Vakuum getrocknet.
   Es wurden 3,75 g des Titelproduktes mit einer Reinheit von 94,1% (nach HPLC), entsprechend einer Ausbeute von 89,8%, bezogen auf 3-Acetoxy-2-cyclobuten-1-on, erhalten.
   Smp: 94°C - 96°C
   - ¹H-NMR: (CDCl₃, 300 MHz) δ in ppm: 1,38 t, J = 9 Hz, 3H
   3,01 q, J = 9 Hz, 2H
   2,90 s, 2H
   4,35 s, 1H
   9,5 b, 2H

### Beispiel 3

### Herstellung von 3-Hydroxy-2-cyclobuten-1-on-cyclohexyldimethylammoniumsalz

a) Zu einer Suspension von 2,1 g 1,3-Cyclobutandion (91,9%; 23,0 mMol) in 40 g Essigester wurde bei 20°C in 15 Min. eine Lösung von 3,21 g N,N-Dimethylcyclohexylamin (99%; 25,0 mMol) in 10 g Essigsäureethylester zugetropft.
   Nach 1 Stunde Rühren bei Raumtemperatur wurde die Suspension abfiltriert und unter Vakuum getrocknet. Es wurden 4,27 g des Titelproduktes mit einer Reinheit von 94,8% (nach HPLC), entsprechend einer Ausbeute von 83,3%, bezogen auf Cyclobutandion, erhalten.
   Smp: 88°C - 90°C
   - ¹H-NMR: (CDCl₃, 300 MHz) in δ ppm: 1,10 - 1,49, m, 5H
   1,65 - 1,80, bd, 1H
   1,88 - 2,01, bd, 2H
   2,04 - 2,13, bd, 2H
   2,78 - 2,91, m, 1H
   2,66, b, 6H
   13,2, b, 1H
b) 3,24 g 3-Acetoxy-2-cyclobuten-1-on (Triketen, 25,0 mMol) wurden in 1,90 g absolutem Ethanol (41,3 mMol) und 40 g Essigester gelöst. Zu dieser Lösung wurden bei 20°C innerhalb von 20 Min. 6,45 g Cyclohexyldimethylamin (99%; 50,0 mMol), gelöst in 10 g Essigsäureethylester, zugetropft.
   Nach 2 Stunden Rühren bei Raumtemperatur wurde die Suspension abfiltriert und unter Vakuum getrocknet.
   Es wurden 4,09 g des Titelproduktes mit einem Gehalt von 94,5% (nach HPLC), entsprechend einer Ausbeute von 73,2%, bezogen auf 3-Acetoxy-2-cyclobuten-1-on (Triketen), erhalten.
   Smp: 91,5°C - 92,4°C

### Beispiel 4

### Herstellung von 3-Hydroxy-2-cyclobuten-1-on-dicyclohexylmethylammoniumsalz

a) Zu einer Suspension von 2,11 g 1,3-Cyclobutandion (23,0 mMol) wurde bei 20°C eine Lösung von 4,99 g Dicyclohexylmethylamin (25,0 mMol) in 10 g Essigsäureethylester zugetropft und 1 Stunde lang gerührt.
   Nach weiteren 30 Min. Rühren bei 5°C wurde die Suspension abfiltriert und unter Vakuum getrocknet.
   Es wurden 5,53 g des Titelproduktes mit einer Reinheit von 94,7% (nach HPLC), entsprechend einer Ausbeute von 81,4%, bezogen auf Cyclobutandion, erhalten.
   Smp: 91,4°C - 92,6°C
b) 3,24 g 3-Acetoxy-2-cyclobuten-1-on (Triketen, 97,5%; 25,0 mMol) wurden in 1,9 g Ethanol (41,3 mMol) und 40 g Essigester gelöst. Zu dieser Lösung wurden bei 20°C in 15 Min. 9,83 g Dicyclohexylmethylamin (98,9%; 50,0 mMol), gelöst in 10 g Essigsäureethylester, zugetropft und 1 Stunde lang bei Raumtemperatur gerührt.
   Nach einer weiteren Stunde Rühren bei 5°C wurde die Suspension abfiltriert und unter Vakuum getrocknet.
   Es wurden 3,21 g des Titelproduktes mit einem Gehalt von 94,5% (nach HPLC), entsprechend einer Ausbeute von 43,5%, bezogen auf 3-Acetoxy-2-cyclobuten-1-on, erhalten.
   Smp: 91,4°C - 92,4°C
   - ¹H-NMR: (CDCl₃, 300 MHz) δ in ppm: 1,09 - 1,42, m, 6H
   1,42 - 1,66, bd, 4H
   1,67 - 1,78, bd, 2H
   1,88 - 2,03, bd, 4H
   2,04 - 2,16, bd, 4H
   2,64, s, 3H
   2,93, b, 2H
   3,13, m, 2H
   4,33, b, 1H

### Beispiel 5

### Herstellung von 3-Hydroxy-2-cyclobuten-1-on-dicyclohexylammoniumsalz

a) Zu einer Suspension von 3,0 g 1,3-Cyclobutandion (97%; 34,6 mMol) in Essigsäureethylester (52,2 ml) wurde bei 20°C in 15 Min. eine Lösung von 7,1 g Dicyclohexylamin (98%; 38,3 mMol) in 15 ml Essigsäureethylester, zugetropft. Nach 1 Stunde Rühren bei Raumtemperatur wurde die Suspension 4 Mal mit Essigsäureethylester gewaschen und unter Vakuum getrocknet. Es wurden 9,4 g des Titelproduktes mit einem Gehalt von 94,7% (nach HPLC), entsprechend einer Ausbeute von 96,7%, bezogen auf Cyclobutandion, erhalten.
   Smp: 188°C - 188,7°C
b) 1000 g Destillationsrückstand der Diketen-Produktion, enthaltend 22,0% 3-Acetoxy-2-cyclobuten-1-on (1,74 Mol), wurden in 1078 ml Essigsäureethylester ( 970 g) gelöst. Zu dieser schwarzen Lösung wurden bei 10°C innerhalb von 60 Min. 707,4 g Dicyclohexylamin (98%; 3,81 Mol) zugetropft. Nach 90 Min. Rühren bei +10°C wurde die Suspension abfiltriert, 3 Mal mit Essigsäureethylester aufgeschlämmt und unter Vakuum getrocknet.
   Es wurden 518 g des Titelproduktes mit einem Gehalt von 65,5% (HPLC), entsprechend einer Ausbeute von 73,4%, bezogen auf 3-Acetoxy-2-cyclobutenon-1-on, erhalten.
   Smp: 169,7°C - 172,4°C
   - Reinigung:: 259 g 3-Hydroxy-2-cyclobuten-1-on-dicyclohexylammoniumsalz (roh; 65,5%; 0,64 Mol) wurden in 207,2 g Eisessig und 310,8 g Essigsäureethylester suspendiert und 2 Stunden bei Raumtemperatur gerührt. Das Produkt wurde 3 Mal mit Essigsäureethylester gewaschen und unter Vakuum getrocknet. Es wurden 176 g des Titelproduktes mit einem Gehalt von 90,2% (nach HPLC), erhalten entsprechend einer Ausbeute von 93,6%.
   Smp: 188°C - 190°C
   - ¹H-NMR: (CD₃OD, 300MHz) δ in ppm: 1,13 - 1,49, m, 10H
   1,66 - 1,80, bd, 2H
   1,80 - 1,97, bd 4H
   2,81, s, 2H
   4,29, s, 1H
   3,09 - 3,27, m, 2H

### Beispiel 6

### Herstellung von 3-Hydroxy-2-cyclobuten-1-on-ammoniumsalz

a) Zu einer Lösung von 2,16 g 1,3-Cyclobutandion (97%; 25,0 mMol) in 25 g Acetonitril wurde bei 20°C innerhalb von 10 Min. eine Lösung von 0,425 g Ammoniakgas (25 Mol) in 25 g Acetonitril zugetropft.
   Nach 30 Min. Rühren bei Raumtemperatur wurde die Suspension abfiltriert, mit Acetonitril (10 ml) gewaschen und unter Vakuum getrocknet. Es wurden 1,48 g des Titelproduktes mit einem Geghalt von 78% (nach HPLC), entsprechend einer Ausbeute von 47,0%, bezogen auf Cyclobutandion, erhalten.
   Smp: 94°C -96°C
b) In eine Lösung von 6,5 g 3-Acetoxy-2-cyclobuten-1-on (Triketen; 97%; 50,0 mMol) in 58,5 g Acetonitril, wurden bei 2°C in 30 Min. 3,1 g Ammoniakgas (182,3 mMol) eingeleitet. Das Reaktionsgemisch wurde weitere 30 Min. bei 2°C gerührt, dann abfiltriert, anschliessend mit Acetonitril (20 ml) gewaschen und unter Vakuum getrocknet.
   Es wurden 4,88 g des Titelproduktes mit einem Gehalt (nach HPLC) von 94%, entsprechend einer Ausbeute von 90,7%, bezogen auf 3-Acetoxy-2-cyclobuten-1-on, erhalten.
   Smp 109°C - 110°C
   - ¹H-NMR: (d₆-DMSO) δ in ppm: 2,49, s, 2H
   3,91, s, 1H
   7,25, b, 4H

### Beispiel 7

### Herstellung von 3-Hydroxy-2-cyclobuten-1-on-natriumsalz

a) Zu einer Lösung von 1,74 g 1,3-Cyclobutandion (97%; 20,0 mMol) in 20 g Acetonitril, wurde bei 20°C in 20 Min. eine Lösung von Natriumethanolat enthaltend 0,506 g Natrium (22 mMol) in 15 g absolutem Ethanol, zugetropft. Das Reaktionsgemisch wurde noch 15 Min. bei Raumtemperatur gerührt, dann abfiltriert und unter Vakuum getrocknet. Es wurden 1,58 g des Titelproduktes mit einer Reinheit von 92,6% (nach HPLC), entsprechend einer Ausbeute von 69,0%, bezogen auf Cyclobutandion, erhalten.
   Smp: > 280°C
   - ¹H-NMR: (d₆-DMSO) δ in ppm: 2,49, s, 2H
   3,92, s, 1H
b) Zu einer Lösung von 3,23 g 3-Acetoxy-2-cyclobuten-1-on (Triketen, 97,5%; 25,0 mMol) in 20 g Acetonitril wurde bei 20°C innerhalb von 15 Min. eine Lösung von 1,099 g Natriumhydroxid (27 mMol) in absolutem Ethanol (10g) zugetropft.
   Nach ca. 2 Stunden Rühren wurde die Suspension bei Raumtemperatur abfiltriert, mit Acetonitril (5 ml) gewaschen und unter Vakuum getrocknet.
   Es wurden 2,38 g des Titelproduktes mit einem Gehalt von 87,2% (nach HPLC), entsprechend einer Ausbeute von 89,8%, bezogen auf 3-Acetoxy-2-cyclobuten-1-on, erhalten.
c) Zu einer Lösung von 28,4 g Destillationsrückstand aus der Diketen-Produktion mit einem Gehalt von 44,4% 3-Acetoxy--2-cyclobuten-1-on (Triketen; 98 mMol) in 250 g Essigsäure-ethylester wurde in 45 Min. bei 10°C eine Lösung von 4,6 g Natriumhydroxid (115 mMol) in 75 g absolutem Ethanol zugetropft. Die Suspension wurde filtriert. Der Rückstand wurde unter Vakuum getrocknet.
Es wurden 17,1 g des Titelproduktes mit einem Gehalt von 46,7% (nach HPLC) entsprechend einer Ausbeute von 75,3%, bezogen auf 3-Acetoxy-2-cyclobuten-1-on, erhalten.

### Beispiel 8

### Herstellung von 3-Hydroxy-2-cyclobuten-1-on-kaliumsalz

Zu einer Lösung von 3,23 g 3-Acetoxy-2-cyclobuten-1-on (Triketen, 97,5%; 25 mMol) in Acetonitril (20 g) wurde bei Raumtemperatur innerhalb 10 Minuten eine Lösung von 1,8 g Kaliumhydroxid (27,5 mMol) in absolutem Ethanol (40 g) zugetropft. Nach 30 Minuten Rühren wurde die Suspension bei Raumtemperatur abfiltriert, mit Acetonitril (5 ml) gewaschen und unter Vakuum getrocknet.
Es wurden 1,99 g des Titelproduktes mit einem Gehalt von 81,5% (nach HPLC), entsprechend einer Ausbeute von 53,1%, bezogen auf 3-Acetoxy-2-cyclobuten-1-on, erhalten.
Smp. > 260°C

### Beispiel 9

### Herstellung von Quadratsäure

a) 3,66 g (50 mMol) 3-Hydroxy-2-cyclobuten-1-on-dicyclohexyl-ammoniumsalz wurden in Methylenchlorid (100 ml) suspendiert und auf -20°C abgekühlt. Dann wurden 10,63 g (150 mMol) Chlorgas eingeleitet. Nach einer Reaktionszeit von 30 Minuten wurde unter Rühren auf Raumtemperatur erwärmt, Methylenchlorid am Rotavapor abdestilliert, und Essisäureethylester (100 ml) zugegeben.
   Der Niederschlag wurde abfiltriert und die Mutterlauge am Rotavapor eingeengt.
   Der so erhaltene Rückstand (11,63 g) wurde mit konzentrierter Schwefelsäure (25 ml) 15 Stunden lang bei 100°C gerührt. Nach Abkühlen im Eisbad wurde der Niederschlag abfiltriert, anschliessend 3 Mal mit Aceton (20 ml) gewaschen und 15 Stunden bei 50°C und 50 mbar getrocknet. Es wurden 1,22 g graues Pulver mit einem Gehalt von 88% Quadratsäure, entsprechend einer Ausbeute von 19%, bezogen auf 3-Hydroxy-2-cyclobuten-1-on-cyclohexylammoniumsalz, erhalten.
b) 73,2 g (1 Mol) 3-Hydroxy-2-cyclobuten-1-on-dicyclohexylammoniumsalz wurden in Essigsäureethylester (1,5 l) suspendiert und anschliessend auf 0°C abgekühlt. Unter Rühren wurden 474,5 g Brom (3 Mol) in Essigsäureethylester (500 ml) hinzugegeben. Der Niederschlag wurde nach 30 Minuten abfiltriert und 2 Mal mit Essigsäureethylester (250 ml) gewaschen. Mutterlauge uns Waschlösungen wurden vereinigt und eingeengt. Es verblieben 442,75 g Rückstand. Anschliessend wurden 415,25 g dieses Rückstandes zu Wasser (34 g) und Schwefelsäure (500 ml) zugegeben. Nach 12 Stunden Rühren bei Raumtemperatur wurde der Niederschlag abfiltriert und 2 Mal mit Aceton (100 ml) gewaschen. Es wurden 111,47 g weisses Titelprodukt mit einem Quadratsäuregehalt von 94,6%, entsprechend einer Ausbeute von 92,45%, bezogen auf eingesetztes 3-Hydroxy-2-cyclobuten-1-on-dicyclohexylammoniumsalz, erhalten.
c) 3,66 g (50 mMol) 3-Hydroxy-2-cyclobuten-1-on-dicyclohexylammoniumsalz wurden in Essigsäureethylester (100 ml) suspendiert. Anschliessend wurden bei Raumtemperatur 20,25 g Sulfurylchlorid (150 mMol) unter Rühren zugetropft.
   Nach einer Reaktionszeit von 1 Stunde wurde mit Essigsäureethylester (50 ml) aufgeschlämmt, dann filtriert, und das Filtrat am Rotavapor eingeengt. Es blieben 11,7 g eines halbflüssigen Rückstandes zurück.
   11,7 g dieses Materials wurden zu konzentrierter Schwefelsäure (25 ml) und Wasser (1,7 g) zugegeben. Nach einer Reaktionszeit von 2 Stunden wurde der Rückstand abfiltriert und 4 Mal mit Aceton (5 ml) gewaschen. Es wurden 1,3 g Titelprodukt mit einem Quadratsäuregehalt von 92,29%, entsprechend einer Ausbeute von 23,14% bezogen auf eingesetztes 3-Hydroxy-2-cyclobuten-1-on-dicyclohexylammoniumsalz, erhalten.
d) 3,66 g (50 mMol) 3-Hydroxy-2-cyclobuten-1-on-dicyclohexylammoniumsalz wurden in Essigsäureethylester (100 ml) suspendiert. Nach Zugabe von 3,95 g Brom (25 mMol) in 20 Minuten wurden 16,87 g Sulfurylchlorid (125 mMol) in 30 Minuten unter Rühren zugetropft.
   Nach einer Reaktionszeit von insgesamt 105 Minuten wurde der Niederschlag 2 Mal mit Essigsäureethylester (50 ml) gewaschen Mutterlauge und Waschlösungen wurden vereinigt und eingeengt.
   Der Rückstand (11,32 g) wurde zu konzentrierter Schwefelsäure (25 ml) und Wasser (1,7 g) bei 100°C zugegeben. Nach einer Reaktionszeit von 2 Stunden wurde der Rückstand abfiltriert und 4 Mal mit Aceton (5 ml) gewaschen. Es wurden 5,26 g weisses Titelprodukt mit einem Quadratsäuregehalt von 92,7%, entsprechend einer Ausbeute von 85,4%, bezogen auf eingesetztes 3-Hydroxy-2-cyclobuten-1-on-dicyclohexylammoniumsalz, erhalten.

### Beispiel 10:

### Herstellung von 1,3-Cyclobutandion ausgehend von 1,3-Cyclobutandion-dicyclohexylammoniumsalz

146,0 g (0,5 Mol) 1,3-Cyclobutandion-dicyclohexylammoniumsalz mit einem Gehalt von 90,9% (nach HPLC) wurden in Acetonitril (1400 ml) suspendiert und auf 10°C abgekühlt. Zu dieser hellbraunen Suspension wurden innerhalb 20 Minuten 21,0 g HCl-Gas (0,573 Mol) eingeleitet. Das Reaktionsgemisch wurde anschliessend noch 30 Minuten lang bei +10°C gerührt und dann filtriert. Anschliessend wurde das Aminhydrochlorid mit Acetonitril (100 ml) gewaschen. Dann wurde im Vakuum bis auf 220 g konzentriert, anschliessend 30 Minuten im Eis-Bad abgekühlt, filtriert, 2 Mal mit Ether (100 ml) gewaschen und bei Raumtemperatur 10 Minuten lang bei 20 mbar getrocknet.
Es wurden 32,07 g des Titelproduktes mit einem Gehalt von 89,8% (nach HPLC), entsprechend einer Ausbeute von 68,5%, bezogen auf Cyclobutandion-Dicyclohexylaminsalz, isoliert.
Smp: 103 -105°C (Zers.)
- ¹H-NMR(d₆-DMSO) δ in ppm: 3,06, s, 2H
4,75, s, 1H
9,5 - 12,5, b, 1H

## Patentansprüche (Patentansprüche für folgende(n) Vertragsstaat(en): AT, BE, CH, DE, DK, FR, GB, IT, LI, LU, NL, SE)

1. 3-Hydroxy-2-cyclobuten-1-on-salze der Formel 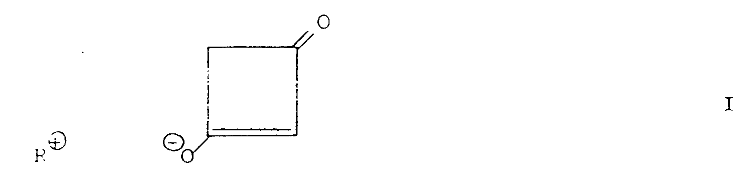 worin R entweder eine Ammonium-Gruppe der allgemeinen Formel 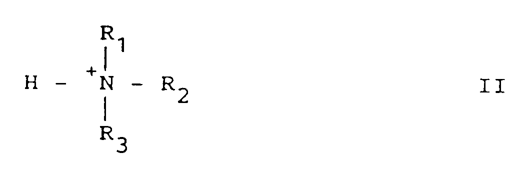 in der R₁, R₂ und R₃ gleichbedeutend oder verschieden sind und ein Wasserstoffatom, eine C₁-C₄-Niederalkyl- oder eine Cycloalkyl-Gruppe darstellen oder worin R ein Alkalimetallatom bedeutet.

2. Verbindung gemäss Anspruch 1, worin in der Ammonium-Gruppe R₁, R₂ und R₃ gleichbedeutend oder verschieden sind und ein Wasserstoffatom, eine C₁ - C₄-Niederalkyl- oder eine Cyclohexylgruppe darstellen.

3. 3-Hydroxy-2-cyclobuten-1-on-Salze gemäss mindestens einem der Patentansprüche 1-2, nämlich
3-Hydroxy-2-cyclobuten-1-on-diethylammoniumsalz,
3-Hydroxy-2-cyclobuten-1-on-cyclohexylammoniumsalz,
3-Hydroxy-2-cyclobuten-1-on-cyclohexyldimethylammoniumsalz,
3-Hydroxy-2-cyclobuten-1-on-dicyclohexylammoniumsalz,
3-Hydroxy-2-cyclobuten-1-on-dicyclohexylmethylammoniumsalz,
3-Hydroxy-2-cyclobuten-1-on-ammoniumsalz,
3-Hydroxy-2-cyclobuten-1-on-natriumsalz oder
3-Hydroxy-2-cyclobuten-1-on-kaliumsalz.

4. Verfahren zur Herstellung von Verbindungen gemäss mindestens einem der Patentansprüche 1-3, dadurch gekennzeichnet, dass man entweder 3-Acetoxy-2-cyclobuten-1-on oder 1,3-Cyclobutandion mit einer Base umsetzt.

5. Verfahren nach Patentanspruch 4, dadurch gekennzeichnet, dass man als Base ein Amin der allgemeinen Formel 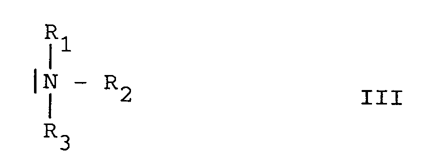 einsetzt, worin R₁, R₂ und R₃ die im Anspruch 1 genannte Bedeutung haben, wobei R₁, R₂ und R₃ gleichbedeutend oder verschieden sind und z.B. ein Wasserstoffatom, eine C₁ - C₄ Niederalkyl- oder eine Cyclohexylgruppe darstellen können.

6. Verfahren nach Patentanspruch 4, dadurch gekennzeichnet, dass man als Base ein Alkalimetallalkoholat oder ein Alkalimetallhydroxid einsetzt.

7. Verfahren nach Patentanspruch 4, dadurch gekennzeichnet, dass man das 3-Acetoxy-2-cyclobuten-1-on in Form eines Destillationsrückstands aus der Diketen-Produktion mit einem Gehalt an 3-Acetoxy-2-cyclobuten-1-on von 5 bis 60 Gew.% einsetzt.

8. Verfahren nach mindestens einem der Patentansprüche 4 bis 7, dadurch gekennzeichnet, dass man die Umsetzung von 3-Acetoxy-2-cyclobuten-1-on oder von 1,3-Cyclobutandion bei einer Temperatur von -40 bis 50°C durchführt.

9. Verwendung der 3-Hydroxy-2-cyclobuten-1-on-salze gemäss Formel I zur Herstellung von Quadratsäure der Formel 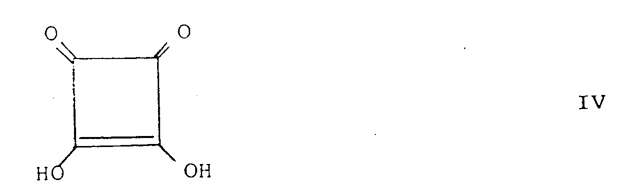 dadurch gekennzeichnet, dass man die 3-Hydroxy-2-cyclobuten-1-on-salze in einer ersten Stufe halogeniert und dann in einer zweiten Stufe zur Quadratsäure hydrolysiert.

10. Verwendung nach Patentanspruch 9, dadurch gekennzeichnet, dass man die Halogenierung mit Brom, Chlor bzw. Sulfurylchlorid, z.B. zuerst mit Brom, dann mit Chlor oder Sulfurylchlorid durchführt.

11. Verwendung nach mindestens einem der Patentansprüche 9 bis 10, dadurch gekennzeichnet, dass man die Halogenierung bei einer Temperatur von -40 bis +40°C durchführt.

12. Verwendung der 3-Hydroxy-2-cyclobuten-1-on-salze nach mindestens einem der Patentansprüche 9 bis 11, dadurch gekennzeichnet, dass man die Hydrolyse mit Mineralsäuren, z.B. Schwefelsäure,mit Sulfonsäuren, mit Carbonsäuren oder mit Wasser durchführt.

13. Verwendung der 3-Hydroxy-2-cyclobuten-1-on-salze nach mindestens einem der Patentansprüche 9 und 12, dadurch gekennzeichnet, dass man die Hydrolyse bei einer Temperatur von 50 bis 150°C durchführt.

## Patentansprüche (Patentansprüche für folgende(n) Vertragsstaat(en): ES)

1. Verfahren zur Herstellung von
3-Hydroxy-2-cyclobuten-1-on-salzen der Formel 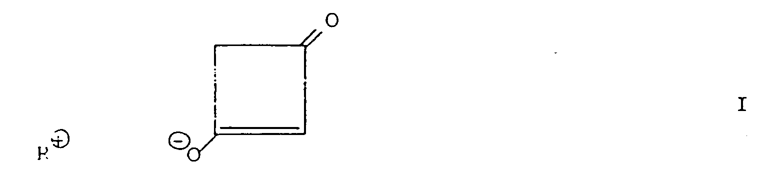 worin R entweder eine Ammonium-Gruppe der allgemeinen Formel 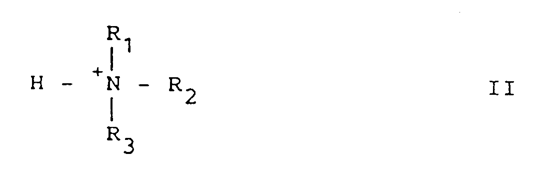 in der R₁, R₂ und R₃ gleichbedeutend oder verschieden sind und ein Wasserstoffatom, eine C₁-C₄-Niederalkyl- oder eine Cycloalkyl-Gruppe darstellen oder worin R ein Alkalimetallatom bedeutet,
dadurch gekennzeichnet, daß man eine Verbindung ausgewählt aus 3-Acetoxy-2-cyclobuten-1-on und 1,3-Cyclobutandion mit einer Base umsetzt.

2. Verfahren gemäß Anspruch 1, dadurch gekennzeichnet, daß in der Ammonium-Gruppe
R₁, R₂ und R₃ gleichbedeutend oder verschieden sind und ein Wasserstoffatom, eine C₁ - C₄-Niederalkyl- oder eine Cyclohexylgruppe darstellen.

3. Verfahren zur Herstellung von
3-Hydroxy-2-cyclobuten-1-on-Salzen gemäß mindestens einem der Patentansprüche 1 bis 2, und zwar von
3-Hydroxy-2-cyclobuten-1-on-diethylammoniumsalz,
3-Hydroxy-2-cyclobuten-1-on-cyclohexylammoniumsalz,
3-Hydroxy-2-cyclobuten-1-on-cyclohexyldimethylammoniumsalz,
3-Hydroxy-2-cyclobuten-1-on-dicyclohexylammoniumsalz,
3-Hydroxy-2-cyclobuten-1-on-dicyclohexylmethylammoniumsalz,
3-Hydroxy-2-cyclobuten-1-on-ammoniumsalz,
3-Hydroxy-2-cyclobuten-1-on-natriumsalz oder
3-Hydroxy-2-cyclobuten-1-on-kaliumsalz.

4. Verfahren nach mindestens einem der Patentansprüche 1 bis 3, dadurch gekennzeichnet, daß man als Base ein Amin der allgemeinen Formel 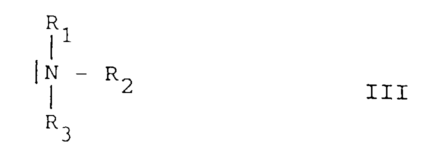 einsetzt, worin R₁, R₂ und R₃ die im Anspruch 1 genannte Bedeutung haben, wobei R₁, R₂ und R₃ gleichbedeutend oder verschieden sind und z.B. ein Wasserstoffatom, eine C₁ - C₄ Niederalkyl- oder eine Cyclohexylgruppe darstellen können.

5. Verfahren nach mindestens einem der Patentansprüche 1 bis 3, dadurch gekennzeichnet, daß man als Base ein Alkalimetallalkoholat oder ein Alkalimetallhydroxid einsetzt.

6. Verfahren nach mindestens einem der Patentansprüche 1 bis 3, dadurch gekennzeichnet, daß man das 3-Acetoxy-2-cyclobuten-1-on in Form eines Destillationsrückstands aus der Diketen-Produktion mit einem Gehalt an 3-Acetoxy-2-cyclobuten-1-on von 5 bis 60 Gew.% einsetzt.

7. Verfahren nach mindestens einem der Patentansprüche 1 bis 6, dadurch gekennzeichnet, daß man die Umsetzung von 3-Acetoxy-2-cyclobuten-1-on oder von 1,3-Cyclobutandion bei einer Temperatur von -40 bis 50°C durchführt.

8. Verfahren zur Herstellung von Quadratsäure der Formel 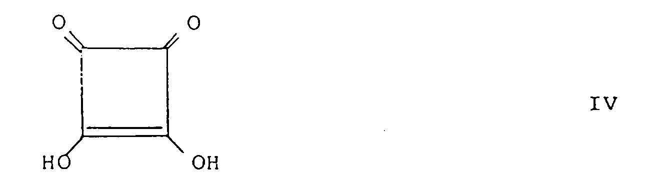 dadurch gekennzeichnet, daß man die 3-Hydroxy-2-cyclobuten-1-on-salze gemäß Formel I in einer ersten Stufe halogeniert und dann in einer zweiten Stufe zur Quadratsäure hydrolysiert.

9. Verfahren nach Patentanspruch 8, dadurch gekennzeichnet, daß man die Halogenierung mit Brom, Chlor bzw. Sulfurylchlorid, z.B. zuerst mit Brom, dann mit Chlor oder Sulfurylchlorid, durchführt.

10. Verfahren nach mindestens einem der Patentansprüche 8 bis 9, dadurch gekennzeichnet, daß man die Halogenierung bei einer Temperatur von -40 bis +40°C durchführt.

11. Verfahren nach mindestens einem der Patentansprüche 8 bis 10, dadurch gekennzeichnet, daß man die Hydrolyse mit Mineralsäuren, z.B. Schwefelsäure, mit Sulfonsäuren, mit Carbonsäuren oder mit Wasser durchführt.

12. Verfahren nach mindestens einem der Patentansprüche 8 bis 11, dadurch gekennzeichnet, daß man die Hydrolyse bei einer Temperatur von 50 bis 150°C durchführt.

## Claims (Claims for the following Contracting State(s): AT, BE, CH, DE, DK, FR, GB, IT, LI, LU, NL, SE)

1. 3-Hydroxy-2-cyclobuten-1-one salts of the formula: 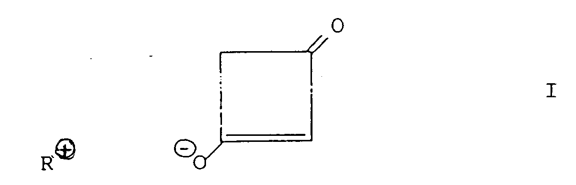 wherein R is either an ammonium group of the general formula: 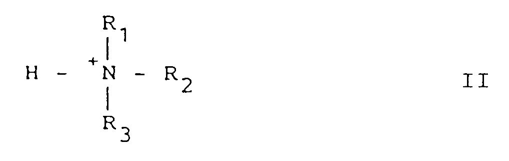 wherein R₁, R₂ and R₃ are equivalent or different, each representing a hydrogen atom, a C₁ to C₄ lower alkyl group or a cycloalkyl group; or wherein R is an alkali metal atom.

2. The compound according to Claim 1, wherein R₁, R₂ and R₃ in the ammonium group are equivalent or different, each representing a hydrogen atom, a C₁ to C₄ lower alkyl group or a cyclohexyl group.

3. 3-Hydroxy-2-cyclobuten-1-one salts according to at least one of Claims 1 and 2, namely
3-hydroxy-2-cyclobuten-1-one diethylammonium salt,
3-hydroxy-2-cyclobuten-1-one cyclohexylammonium salt,
3-hydroxy-2-cyclobuten-1-one cyclohexyldimethylammonium salt,
3-hydroxy-2-cyclobuten-1-one dicyclohexylammonium salt,
3-hydroxy-2-cyclobuten-1-one dicyclohexylmethylammonium salt,
3-hydroxy-2-cyclobuten-1-one ammonium salt,
3-hydroxy-2-cyclobuten-1-one sodium salt, or
3-hydroxy-2-cyclobuten-1-one potassium salt.

4. A process for the preparation of the compounds according to at least one of Claim 1 to 3, characterized in that either 3-acetoxy-2-cyclobuten-1-one or 1,3-cyclobutanedione is reacted with a base.

5. The process according to Claim 4, characterized by using as a base an amine of the general formula: 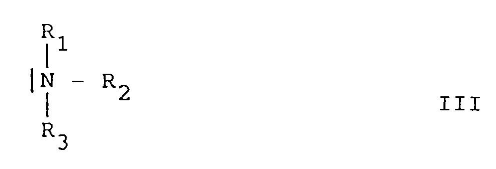 wherein R₁, R₂ and R₃ have the meaning set forth in Claim 1 according to which R₁, R₂ and R₃ are equivalent or different, representing e.g. a hydrogen atom, a C₁ to C₄ lower alkyl group, or a cyclohexyl group.

6. The process according to Claim 4, characterized by using as a base an alkali metal alcoholate or an alkali metal hydroxide.

7. The process according to Claim 4, characterized in that the 3-acetoxy-2-cyclobuten-1-one is used in form of a distillation residue from diketene production having a content in 3-acetoxy-2-cyclobuten-1-one of from 5 to 60% by weight.

8. The process according to at least one of Claims 4 to 7, characterized in that the reaction of 3-acetoxy-2-cyclobuten-1-one or of 1,3-cyclobutanedione is conducted at a temperature of from -40 to 50°C.

9. Use of the 3-hydroxy-2-cyclobuten-1-one salts according to formula I for the preparation of squaric acid of the formula: 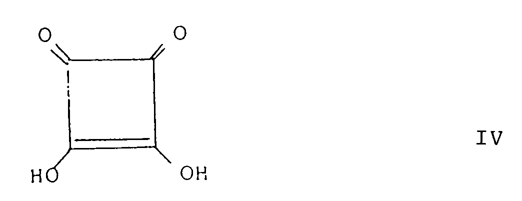 characterized in that the 3-hydroxy-2-cyclobuten-1-one salts are halogenated in a first step and then hydrolized to squaric acid in a second step.

10. Use according to Claim 9, characterized in that halogenation is conducted with bromine, chlorine and, respectively, sulfuryl chloride, e.g. first with bromine and then with chlorine or sulfuryl chloride.

11. Use according to at least one of Claims 9 and 10, characterized in that halogenation is conducted at a temperature of from -40 to +40°C.

12. Use of the 3-hydroxy-2-cyclobuten-1-one salts according to at least one of Claims 9 to 11, characterized in that hydrolysis is conducted with mineral acids such as sulfuric acid, with sulfonic acids, with carboxylic acids, or with water.

13. Use of the 3-hydroxy-2-cyclobuten-1-one salts according to at least one of Claims 9 and 12, characterized in that hydrolysis is conducted at a temperature of from 50 to 150°C.

## Claims (Claims for the following Contracting State(s): ES)

1. A process for the preparation of 3-hydroxy-2-cyclobuten-1-one of the formula: 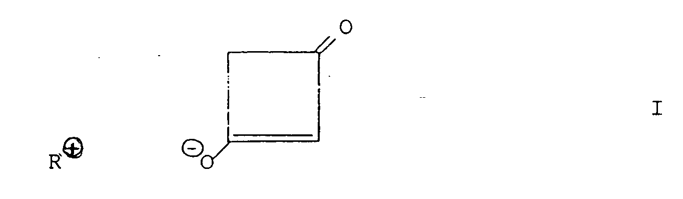 wherein R either is an ammonium group of the general formula: 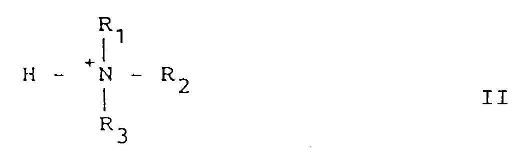 wherein R₁, R₂ and R₃ are equivalent or different, each representing a hydrogen atom, a C₁ to C₄ lower alkyl group or a cycloalkyl group; or wherein R is an alkali metal atom; characterized in that a compound selected from 3-acetoxy-2-cyclobuten-1-one and 1,3-cyclobutanedione is reacted with a base.

2. The process according to Claim 1, characterized in that R₁, R₂ and R₃ in the ammonium group are equivalent or different, each representing a hydrogen atom, a C₁ to C₄ lower alkyl group or a cyclohexyl group.

3. The process for the preparation of 3-hydroxy-2-cyclobuten-1-one salts according to at least one of Claim 1 and 2, namely of
3-hydroxy-2-cyclobuten-1-one diethylammonium salt,
3-hydroxy-2-cyclobuten-1-one cyclohexylammonium salt,
3-hydroxy-2-cyclobuten-1-one cyclohexyldimethylammonium salt,
3-hydroxy-2-cyclobuten-1-one dicyclohexylammonium salt,
3-hydroxy-2-cyclobuten-1-one dicyclohexylmethylammonium salt,
3-hydroxy-2-cyclobuten-1-one ammonium salt,
3-hydroxy-2-cyclobuten-1-one sodium salt, or
3-hydroxy-2-cyclobuten-1-one potassium salt.

4. The process according to at least one of Claims 1 to 3, characterized by using as a base an amine of the general formula: 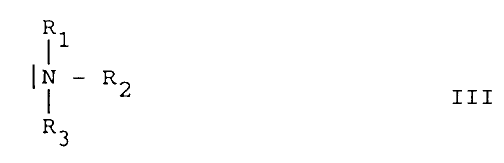 wherein R₁, R₂ and R₃ have the meaning set forth in Claim 1 according to which R₁, R₂ and R₃ are equivalent or different, representing e.g. a hydrogen atom, a C₁ to C₄ lower alkyl group, or a cyclohexyl group.

5. The process according to at least one of Claims 1 to 3, characterized by using as a base an alkali metal alcoholate or an alkali metal hydroxide.

6. The process according to at least one of Claims 1 to 3, characterized in that the 3-acetoxy-2-cyclobuten-1-one is used in form of a distillation residue from diketene production having a content in 3-acetoxy-2-cyclobuten-1-one of from 5 to 60% by weight.

7. The process according to at least one of Claims 1 to 6, characterized in that reaction of 3-acetoxy-2-cyclobuten-1-one or of 1,3-cyclobutanedione is conducted at a temperature of from -40 to 50°C.

8. A process for the preparation of squaric acid of the formula: 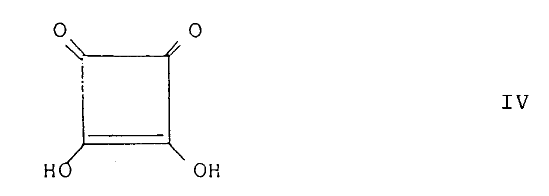 characterized in that the 3-hydroxy-2-cyclobuten-1-one salts according to formula 1 are halogenated in a first step and then hydrolized in a second step to squaric acid.

9. The process according to Claim 8, characterized in that halogenation is conducted with bromine, chlorine and, respectively, sulfuryl chloride, e.g. first with bromine and then with chlorine or sulfuryl chloride.

10. The process according to at least one of Claims 8 and 9, characterized in that halogenation is conducted at a temperature of from -40 to +40°C.

11. The process according to at least one of Claims 8 to 10, characterized in that hydrolysis is conducted with mineral acids such as sulfuric acid, with sulfonic acids, with carboxylic acids, or with water.

12. The process according to at least one of Claims 8 to 11, characterized in that hydrolysis is conducted at a temperature of from 50 to 150°C.

## Revendications (Revendications pour l'(les) Etat(s) contractant(s) suivant(s): AT, BE, CH, DE, DK, FR, GB, IT, LI, LU, NL, SE)

1. Sels de la 3-hydroxy-2-cyclobutén-1-one de formule 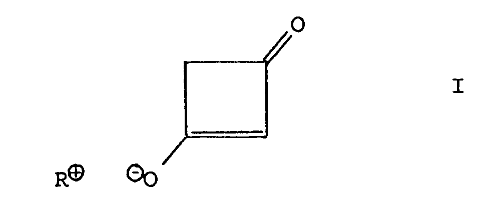 dans laquelle R représente un groupe ammonium de formule générale 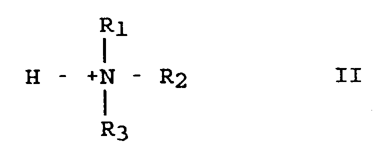 où R₁, R₂ et R₃ sont identiques ou différents et représentent un atome d'hydrogène, un groupe alkyle inférieur en C₁-C₄ ou un groupe cycloalkyle, ou dans laquelle R représente un atome de métal alcalin.

2. Composé selon la revendication 1, dans lequel, dans le groupe ammonium, R₁, R₂ et R₃ sont identiques ou différents et représentent un atome d'hydrogène, un groupe alkyle inférieur en C₁-C₄ ou un groupe cyclohexyle.

3. Sels de la 3-hydroxy-2-cyclobutén-1-one selon au moins l'une des revendications 1 et 2, à savoir
le sel de diéthylammonium de la 3-hydroxy-2-cyclobutén-1-one,
le sel de cyclohexylammonium de la 3-hydroxy-2-cyclobutén-1-one,
le sel de cyclohexyldiméthylammonium de la 3-hydroxy-2-cyclobutén-1-one,
le sel de dicyclohexylammonium de la 3-hydroxy-2-cyclobutén-1-one,
le sel de dicyclohexylméthylammonium de la 3-hydroxy-2-cyclobutén-1-one,
le sel d'ammonium de la 3-hydroxy-2-cyclobutén-1-one,
le sel de sodium de la 3-hydroxy-2-cyclobutén-1-one ou
le sel de potassium de la 3-hydroxy-2-cyclobutén-1-one.

4. Procédé de préparation de composés selon au moins l'une des revendications 1 à 3, caractérisé en ce que l'on fait réagir la 3-acétoxy-2-cyclobutén-1-one ou la 1,3-cyclobutanedione avec une base.

5. Procédé selon la revendication 4, caractérisé en ce que l'on utilise comme base une amine de formule générale 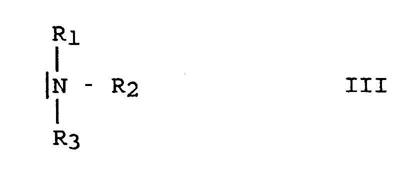 dans laquelle R₁, R₂ et R₃ ont la signification donnée dans la revendication 1, R₁, R₂ et R₃ étant identiques ou différents et pouvant représenter par exemple un atome d'hydrogène, un groupe alkyle inférieur en C₁-C₄ ou un groupe cyclohexyle.

6. Procédé selon la revendication 4, caractérisé en ce que l'on utilise comme base un alcoolate de métal alcalin ou un hydroxyde de métal alcalin.

7. Procédé selon la revendication 4, caractérisé en ce que l'on utilise la 3-acétoxy-2-cyclobutén-1-one sous forme d'un résidu de distillation provenant de la production de dicétène à teneur en 3-acétoxy-2-cyclobutén-1-one de 5 à 60% en poids.

8. Procédé selon au moins l'une des revendications 4 à 7, caractérisé en ce que l'on conduit la réaction de la 3-acétoxy-2-cyclobutén-1-one ou de la 1,3-cyclobutanedione à une température de -40 à 50°C.

9. Utilisation des sels de 3-hydroxy-2-cyclobutén-1-one selon la formule I pour la préparation d'acide squarique de formule 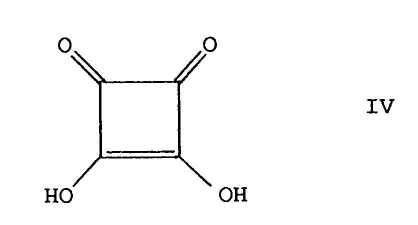 caractérisée en ce que l'on halogène les sels de 3-hydroxy-2-cyclobutén-1-one dans une première étape puis on les hydrolyse en acide squarique dans une deuxième étape.

10. Utilisation selon la revendication 9, caractérisée en ce que l'on conduit l'halogénation avec le brome, le chlore ou le chlorure de sulfuryie, par exemple d'abord avec le brome puis avec le chlore ou le chlorure de sulfuryle.

11. Utilisation selon au moins l'une des revendications 9 et 10, caractérisée en ce que l'on conduit l'halogénation à une température de -40 à +40°C.

12. Utilisation des sels de 3-hydroxy-2-cyclobutén-1-one selon au moins l'une des revendications 9 à 11, caractérisée en ce que l'on conduit l'hydrolyse avec des acides minéraux, par exemple l'acide sulfurique, avec des acides sulfoniques, avec des acides carboxyliques ou avec l'eau.

13. Utilisation des sels de 3-hydroxy-2-cyclobutén-1-one selon au moins l'une des revendications 9 à 12, caractérisée en ce que l'on conduit l'hydrolyse à une température de 50 à 150°C.

## Revendications (Revendications pour l'(les) Etat(s) contractant(s) suivant(s): ES)

1. Procédé de préparation de sels de la 3-hydroxy-2-cvclobutén-1-one de formule 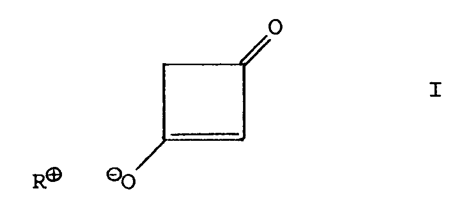 dans laquelle R représente un groupe ammonium de formule générale 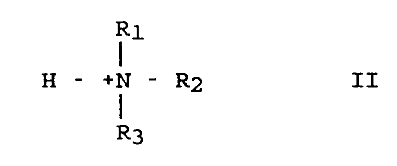 où R₁, R₂ et R₃ sont identiques ou différents et représentent un atome d'hydrogène, un groupe alkyle inférieur en C₁-C₄ ou un groupe cycloalkyle, ou dans laquelle R représente un atome de métal alcalin, caractérisé en ce que l'on fait réagir un composé choisi parmi la 3-acétoxy-2-cyclobutén-1-one ou la 1,3-cyclobutanedione avec une base.

2. Procédé selon la revendication 1, caractérisé en ce que, dans le groupe ammonium, R₁, R₂ et R₃ sont identiques ou différents et représentent un atome d'hydrogène, un groupe alkyle inférieur en C₁-C₄ ou un groupe cyclohexyle.

3. Procédé de préparation de sels de la 3-hydroxy-2-cyclobutén-1-one selon au moins l'une des revendications 1 et 2, à savoir
du sel de diéthylammonium de la 3-hydroxy-2-cyclobutén-1-one,
du sel de cyclohexylammonium de la 3-hydroxy-2-cyclobutén-1-one,
du sel de cyclohexyldiméthylammonium de la 3-hydroxy-2-cyclobutén-1-one,
du sel de dicyclohexylammonium de la 3-hydroxy-2-cyclobutén-1-one,
du sel de dicyclohexylméthylammonium de la 3-hydroxy-2-cyclobutén-1-one,
du sel d'ammonium de la 3-hydroxy-2-cyclobutén-1-one,
du sel de sodium de la 3-hydroxy-2-cyclobutén-1-one ou
du sel de potassium de la 3-hydroxy-2-cyclobutén-1-one.

4. Procédé selon au moins l'une des revendications 1 à 3, caractérisé en ce que l'on utilise comme base une amine de formule générale 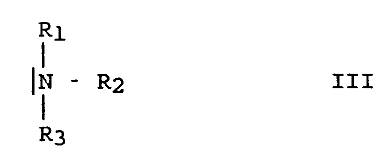 dans laquelle R₁, R₂ et R₃ ont la signification donnée dans la revendication 1, R₁, R₂ et R₃ étant identiques ou différents et pouvant représenter par exemple un atome d'hydrogène, un groupe alkyle inférieur en C₁-C₄ ou un groupe cyclohexyle.

5. Procédé selon au moins l'une des revendications 1 à 3, caractérisé en ce que l'on utilise comme base un alcoolate de métal alcalin ou un hydroxyde de métal alcalin.

6. Procédé selon au moins l'une des revendications 1 à 3, caractérisé en ce que l'on utilise la 3-acétoxy-2-cyclobutén-1-one sous forme d'un résidu de distillation provenant de la production de dicétène à teneur en 3-acétoxy-2-cyclobutén-1-one de 5 à 60% en poids.

7. Procédé selon au moins l'une des revendications 1 à 6, caractérisé en ce que l'on conduit la réaction de la 3-acétoxy-2-cyclobutén-1-one ou de la 1,3-cyclobutanedione à une température de -40 à 50°C.

8. Procédé de préparation d'acide squarique de formule 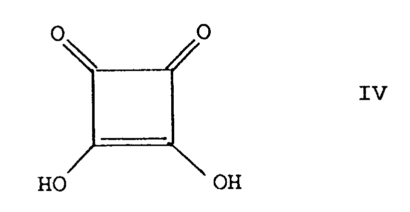 caractérisé en ce que l'on halogène les sels de 3-hydroxy-2-cyclobutén-1-one selon la formule I dans une première étape puis on les hydrolyse en acide squarique dans une deuxième étape.

9. Procédé selon la revendication 8, caractérisé en ce que l'on conduit l'halogénation avec le brome, le chlore ou le chlorure de sulfuryle, par exemple d'abord avec le brome puis avec le chlore ou le chlorure de sulfuryle.

10. Procédé selon au moins l'une des revendications 8 et 9, caractérisé en ce que l'on conduit l'halogénation à une température de -40 à +40°C.

11. Procédé selon au moins l'une des revendications 8 à 10, caractérisé en ce que l'on conduit l'hydrolyse avec des acides minéraux, par exemple l'acide sulfurique, avec des acides sulfoniques, avec des acides carboxyliques ou avec l'eau.

12. Procédé selon au moins l'une des revendications 8 à 11, caractérisé en ce que l'on conduit l'hydrolyse à une température de 50 à 150°C.
